# EUROPEAN PATENT APPLICATION

(11) **EP 0 949 228 A1**
(43) Date of publication of application: **13.10.1999**
(21) Application number: 99201054.6
(22) Date of filing: 31.03.1999
(51) Int. Cl.: C07C 2/82

(54) **Process for the conversion of methane to higher hydrocarbons**

(30) Priority: 07.04.1998 IT MI980733
(71) Applicant: ENI S.p.A., 00144 Roma (IT)
(72) Inventor: Belmonte, Giuseppe, 20098 San Giuliano Milanese, Milan (IT); Molinari, Mario, 20133 Milan (IT); Schwarz, Peter, 20144 Milano (IT)
(74) Representative: De Gregori, Antonella

(57) **Abstract**

Process for the conversion of methane to higher hydrocarbons which comprises the following operating steps:
a) pyrolizing methane in the presence of oxygen at a temperature ranging from 1000 to 1200°C in a reaction apparatus maintained at 30-40 bars;
b) rapidly cooling the pyrolysis mixture by expansion of the gaseous phase, immediately after the reaction;
c) recovering the C₂-C₃ hydrocarbons generated during step (a), from the reaction mixture by distillation.

## Description

The present invention relates to a process for the conversion of methane to higher hydrocarbons.

More specifically, the present invention relates to a process in homogeneous phase for the conversion of methane to ethylene and propylene.

The oxidative coupling reaction in homogeneous phase of methane for the production of hydrocarbons such as ethane/ethylene and propane/propylene, is known in scientific literature. For example, Industrial & Engineering Chemistry Research, 1990, 29, 2-10, describes the conversion of methane, mainly to ethane and ethylene and to a lesser degree to C₃, by reaction with oxygen at a high pressure and temperatures exceeding 1000 K.

Analogously, the American Institute of Chemical Engineers Journal, vol. 40, 3, 521-535, describes the oxidative coupling of methane carried out without catalysts operating at pressures of up to 10 bars and a temperatures higher than 1000 K.

A typical characteristic of these reactions is that they give significant selectivities to useful products, such as ethylene and propylene, only at high temperatures and above all operating at a high pressure.

In this type of reaction however, carbon and hydrogen are thermodynamically favoured with respect to the methane and hydrocarbons produced. This means that to maintain a satisfactory production of hydrocarbons it is necessary to block these oxidative coupling reactions by rapid cooling of the reagent mixture.

The Applicant has now found a process for effecting the oxidative coupling reaction of methane which overcomes the problem of the known art, as it obtains the cooling of the reaction mixture in very short times by rapid expansion of the gaseous phase, preferably in a turbine, with the recovery of energy.

The present invention therefore relates to a process for the conversion of methane to higher hydrocarbons, for example C₂-C₃ hydrocarbons, which comprises the following operating steps:
a) pyrolizing methane in the presence of oxygen at a temperature ranging from 1000 to 1200°C in a reaction apparatus maintained at 30-40 bars;
b) rapidly cooling the pyrolysis mixture by expansion of the gaseous phase, immediately after the reaction;
c) recovering the C₂-C₃ hydrocarbons generated during step (a), from the reaction mixture by distillation.

According to the present invention, operating under the above conditions and with molar ratios CH₄/O₂ ranging from 5 to 8, it is possible to obtain a selectivity to ethylene and propylene of 30-40%.

The rapid cooling of the reaction gases, by expansion of these, brings them to a temperature which slows down the cracking kinetics of the hydrocarbon products of the oxidative coupling of methane. The expansion generally brings the temperature of the reaction gases to values ranging from 500 to 600°C.

The reaction gases are subsequently cooled in a heat exchanger, with the production of water vapor, to a temperature of about 300-400°C. The reaction gases are cooled to room temperature in other heat exchangers. Part of the water formed during the reaction is condensed in this phase.

According to the process of the present invention, it is preferable to carry out the expansion in a turbine, with the possibility of recuperating mechanical work.

In addition, as decomposition reactions also of the useful products can be activated at the reaction temperatures, it is preferable for the expansion of the gases to be carried out immediately after the pyrolysis reaction in order to have residence times in reaction of approximately tenths of a second. To obtain this result, it is possible to effect the oxidative coupling reaction inside the combustion chamber of a gas turbine.

The recovery of the C₂-C₃ hydrocarbons from the reaction mixture involves the following operating phases.

With one or more compression steps, the gases are brought to a pressure of 30-40 bars. With each compression step there is a cooling step so as to promote, by condensation, the elimination of the rest of the water formed in the reaction phase.

The gases, essentially anhydrous, are sent to an elimination section of the CO₂ consisting of an absorption column in which an absorbing liquid, for example an aqueous solution at 20% by weight of diethanolamine, is put into close contact with the synthesis gases. Downstream of this section, the gases undergo a finishing treatment, for example, by adsorption with molecular sieves, by eliminating the traces of CO₂ and H₂O still present.

After the elimination of the carbon dioxide and residual water, the gases are cooled with a refrigerating cycle to about -150°C and sent to a first demethanizing column.

The reaction by-products are discharged from the head of the demethanizing column, the non-reacted methane from a lateral slit, recycled to the pyrolysis, and the C₂ ⁺ hydrocarbons from the tail. The latter are subsequently sent to a recovery section of ethylene and propylene, "polymer grade", after hydrogenation of the acetylenes present.

The process for the conversion of methane to higher hydrocarbons of the present invention can be more easily understood by referring to the scheme of the enclosed figure which represents an illustrative and non-limiting embodiment.

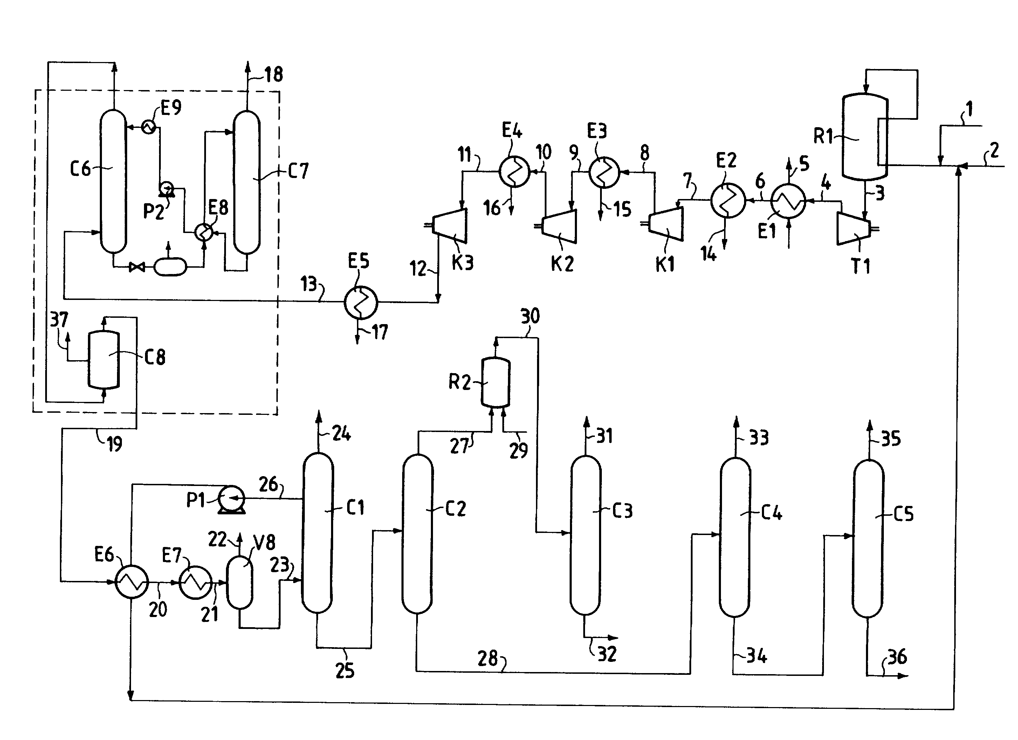

With reference to figure 1, the fresh methane of the charge (1), together with the recycled methane (26), are fed to the pyrolysis reactor (R1). The oxygen coming from the air fractionation plant, not illustrated in the figure, is fed to the same reactor by means of (2), already at the operating pressure of R1.

The reaction products (3), leaving the pyrolysis reactor, are rapidly cooled by expansion in the gas turbine (T1), with the production of energy. The gases discharged from the turbine by means of (4), still hot, are used in the heat exchanger (E1) to produce vapor (5) destined to produce energy in a vapor plant not illustrated in the figure.

The gases are sent from the exchanger (E1), by means of lines (6)-(12) to the coolers (E2)-(E5), alternated with the compressors (K1)-(K3), where the reaction water discharged with lines (14)-(17) is condensed.

The dehumidified gases are then sent, by means of line (13) to the carbon dioxide elimination section comprising two columns (C6) and (C7). The first column, (C6), is a column for the absorption of CO₂ whereas the second column, (C7), is a column for the regeneration of the absorbing liquid. The CO₂ is discharged from the head of (C7) by means of line (18). The gases leaving the absorption column are treated again in the column (C8), operating with molecular sieves, to eliminate further impurities (37).

After elimination of the water, carbon dioxide and other impurities, the reaction gases are sent, by means of line (19), to the exchanger (E6). The gases are then sent to the condenser (E7), by means of line (20), and then to the separation system (V8) by means of line (21).

The non-condensable residues are discharged by means of (22) whereas the condensed phase is fed, with line (23) to a first distillation column (C1) (demethanizer) from whose head additional uncondensable products are discharged, with line (24) and from the tail, a stream (25) essentially consisting of C₂-C₃. A stream consisting of non-reacted methane is removed from a lateral slit (26), which, after frigories exchange in (E6), is recycled to the pyrolysis.

The uncondensable products of streams (22) and (24) essentially consist of non-reacted methane, carbon dioxide and hydrogen. This mixture therefore has a high heating value and can be used to produce energy.

The liquid stream (25) is sent to a second distillation column (C2) (de-ethanizer) from whose head a stream, (27), essentially consisting of C₂, is recovered, together with a stream from the bottom (28) essentially consisting of C₃.

The stream (27) is fed to the reactor (R2) for the hydrogenation of the acetylenes and then, as stream (30) to the distillation column (C3) to obtain ethylene (31), "polymer grade" at the head, and ethane (32) at the tail.

The stream (28) goes to the distillation column (C4) (depropanizer) from whose head the C₂ still present (33) are discharged, together with the propane/propylene mixture (34) from the tail. The latter goes to the distillation column (C5) to produce, at the head (36), propylene "polymer grade" and propane at the tail.

An illustrative but non-limiting example is provided hereunder for a better understanding of the present invention and for its embodiment.

### EXAMPLE

The operation is carried out in accordance with the scheme of the enclosed figure relating to a plant having the potentiality of about 1.4 Kg/day of ethylene.

The material balance is indicated in Tables 1-3 below.

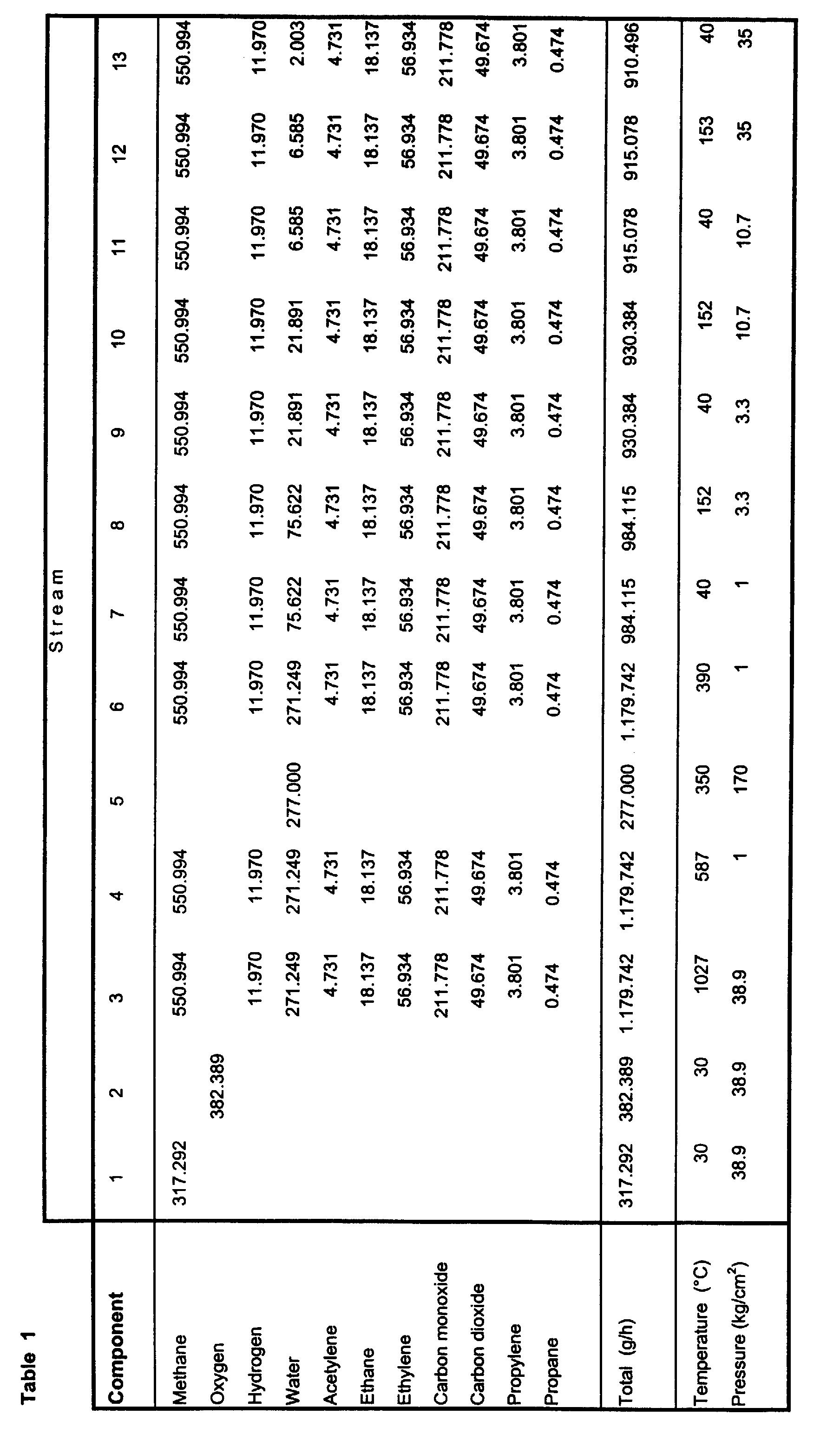

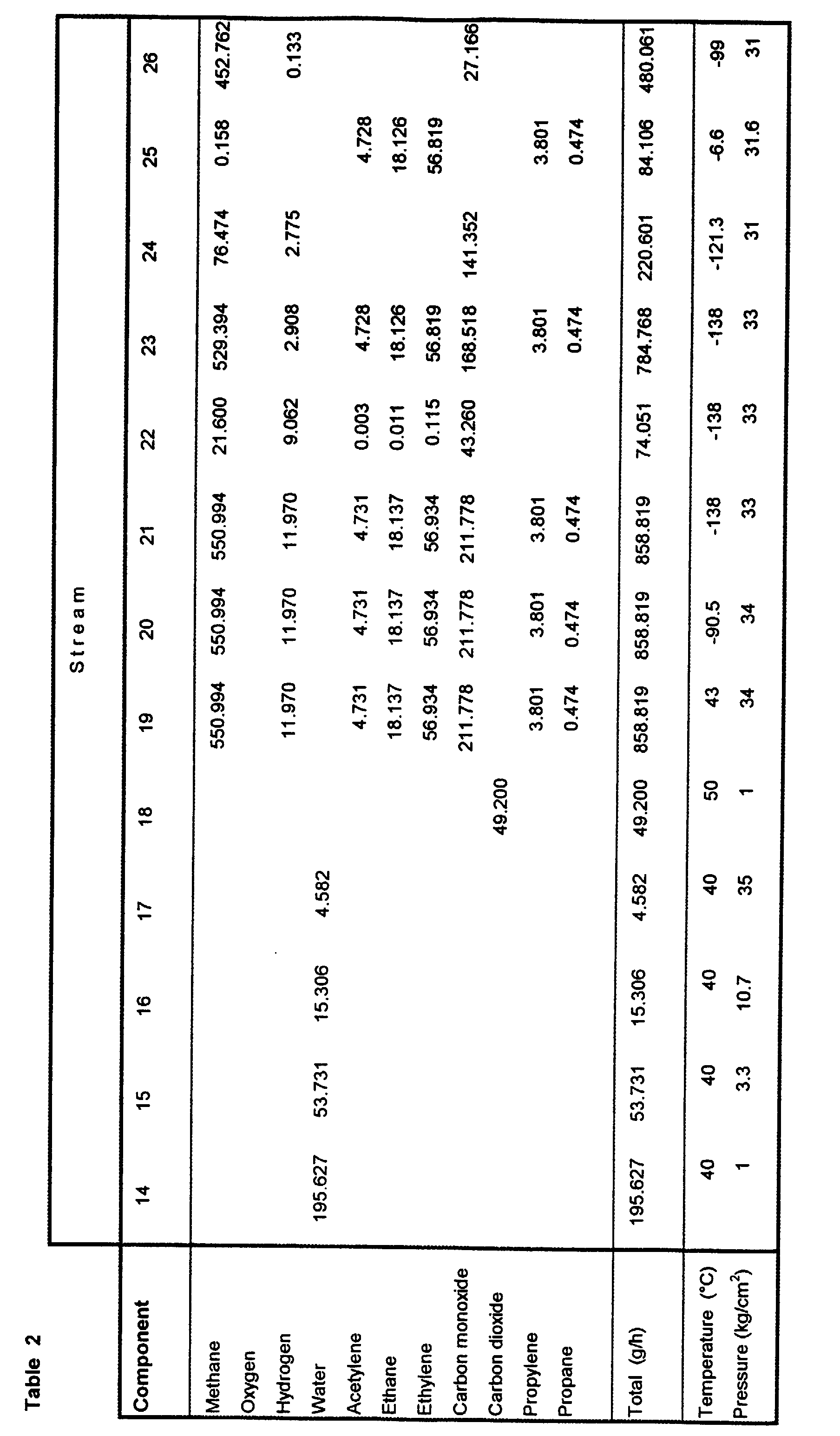

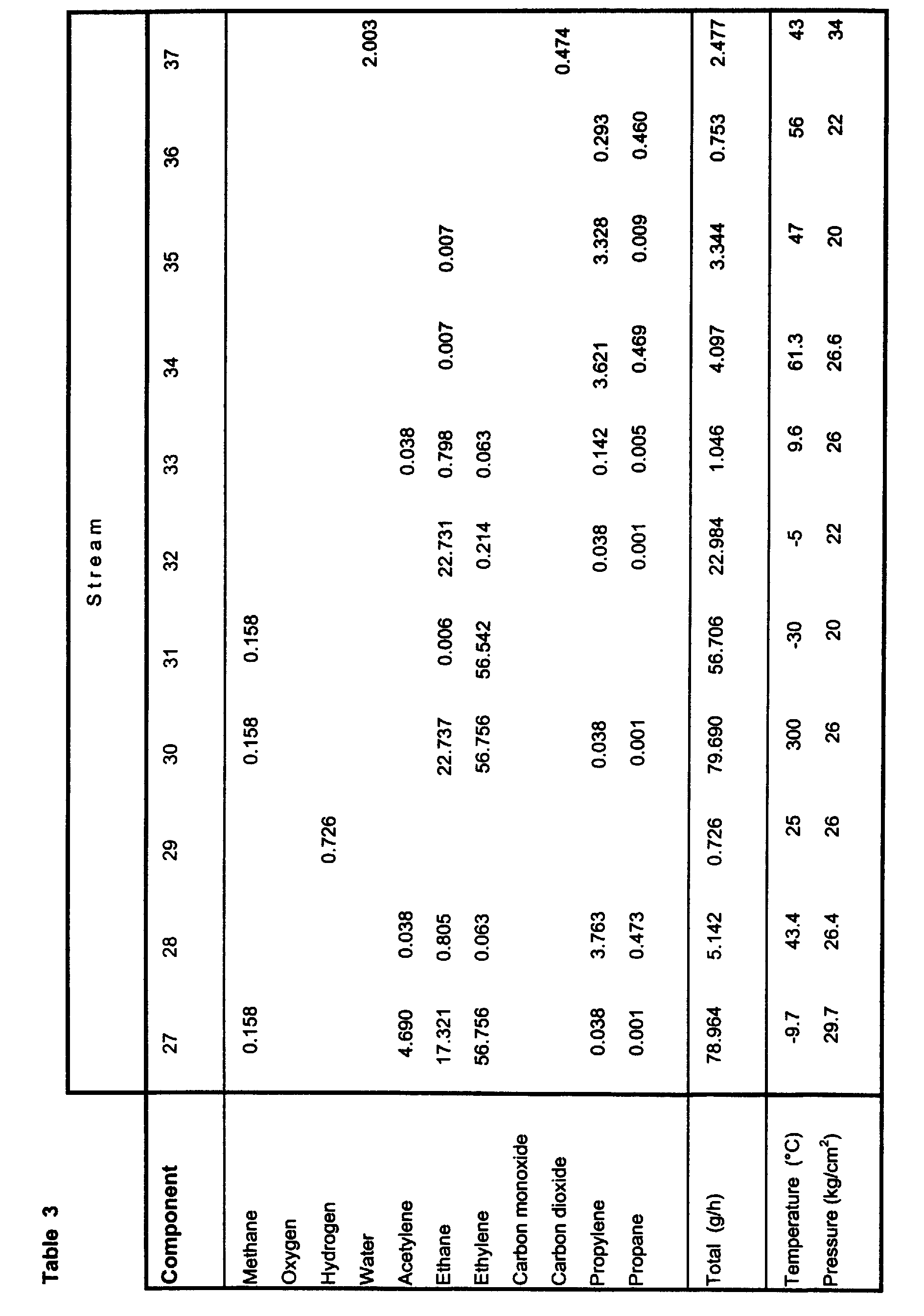

## Claims

1. A process for the conversion of methane to higher hydrocarbons which comprises the following operating steps:
a) pyrolizing methane in the presence of oxygen at a temperature ranging from 1000 to 1200°C in a reaction apparatus maintained at 30-40 bars;
b) rapidly cooling the pyrolysis mixture by expansion of the gaseous phase, immediately after the reaction;
c) recovering the C₂-C₃ hydrocarbons generated during step (a), from the reaction mixture by distillation.

2. The process according to claim 1, wherein the higher hydrocarbons are C₂-C₃ hydrocarbons.

3. The process according to claim 1 or 2, wherein the molar ratios CH₄/O₂ are between 5 and 8.

4. The process according to any of the previous claims, wherein the rapid cooling of the reaction gases, by expansion of these, takes place in a gas turbine.

5. The process according to any of the previous claims, wherein the temperature of the gas, after expansion, is between 500 and 600°C.
